# EUROPEAN PATENT APPLICATION

(11) **EP 1 950 289 A1**
(43) Date of publication of application: **30.07.2008**
(21) Application number: 06798505.1
(22) Date of filing: 03.10.2006
(51) Int. Cl.: C12N 15/00, C12N 15/09

(54) **METHOD OF COLLECTING DNA FROM BIOLOGICAL SAMPLE**

(30) Priority: 03.10.2005 JP 2005289698; 31.03.2006 JP 2006098286
(71) Applicant: National University Corporation Shinshu University, Matsumoto-shi, Nagano 3908621 (JP); HITACHI SOFTWARE ENGINEERING CO., LTD., Shinagawa-ku 140-0002 Tokyo (JP)
(72) Inventor: FUKUSHIMA, Hirohumi, Nagano 390-8621 (JP); NAGASAKI, Kanako, Tokyo 140-0002 (JP)
(74) Representative: Jönsson, Hans-Peter
(86) International application number: PCT/JP2006/320155
(87) International publication number: WO 2007/043494

(57) **Abstract**

A method in which DNA is extracted from a precious sample of organism simply and efficiently without being contaminated by impurities is provided. The method for extracting DNA comprises of:
1) a process for pretreatment having;
a procedure for obtaining fragments by using a whole sample of organism of hard tissue, by cutting the sample, or by crushing the sample, and
a subsequent procedure of decalcification by adding an EDTA aqueous solution thereto,
2) a process for dissolution having;
a procedure for adding a buffer solution of a solubilizer, which dissolves a complex of DNA-protein in the sample of organism, and a proteolytic enzyme to a solution of (1) to prepare a solution of DNA,
3) a process for extraction having;
a procedure for adding a saturated phenol buffer including tris(hydroxymethyl)aminomethane hydrochloride or an aqueous solution including sodium iodide and isopropanol to the solution of the complex of (2) for deproteinization, and then separating an extract of DNA,
4) a process for purification having;
a subsequent process for purification of DNA by passing the extract of DNA of (3) through a column.

## Description

### Technical Field

This invention relates to a method for extracting DNA from a sample of organism carried out in cases of forensic medical DNA (deoxyribonucleic acid) analysis and biological genetic research.

### Background Art

A forensic DNA analysis is performed to identify a relationship between a parent and a child or between siblings by using living tissues and also to identify tissue of decedent of an affair or an accident, or of old remains of war dead. It is also used for gathering evidences for identifying a suspect by using things left at a crime scene such as hair, body fluid, body fluid stain and so on. And a DNA analysis is performed for biological genetic research such as sequencing, genetic engineering or molecular biology in regard to humans, plants and animals.

In these analyses, DNA is extracted from a sample of organism such as living or dead human tissue illustrated with hard tissue (ex. tooth, bone, nail), soft tissue (ex. hair, skin, muscle, internal organ, vascular), body fluid (ex. blood, lymph, saliva, semen, urine), and body fluid stain, and living or dead plant or animal tissue.

Prior methods for extracting DNA from a tooth or a bone are commonly performed as follows. A tooth or a bone is washed with an aqueous solution of sodium hypochloride, water, and then ethanol to remove contaminant or adherents from the surface thereof. After being dried and powdered, it is decalcified by an aqueous solution of ethylenediamine tetraacetic acid (EDTA) for many hours and then dissolved by a resolvable solution and proteolytic enzyme. After that, DNA is extracted with mixture of phenol-chloroform-isoamylalcohol, precipitated by ethanol from aqueous phase thereof, and purified to obtain extractive DNA.

In Japanese Patent Provisional Publication No. 2004-201525, a simple method for extracting DNA is described. In the method, after DNA extracting agent was added to a powdered sample of organism, the sample was precipitated by ethanol and purified to obtain extractive DNA.

"Forensic Sciences, 44(3), p.264-272, (2003)" mentions that for identifying victims of the attacks on the World Trade Center Towers in United States on September 11, 2001, short tandem repeat (STR) 13-locus was detected from degraded DNA after a tooth or a bone was powdered by prior methods.

Generally, when a sample for DNA is powdered, it is contaminated with DNA from undesired tissue. When the sample is decalcified by EDTA for many hours, DNA is decomposed. And when the sample is not deproteinized or is old, impurity such as protein, which obstructs the DNA analysis, contaminates the desired DNA. In addition, when DNA is precipitated by ethanol, the precious DNA is lost.

### Disclosure of Invention

The present invention has been developed to solve the foregoing problems. It is an object of the present invention to provide a method in which DNA is extracted from a precious sample of organism simply and efficiently without being contaminated by impurities.

A method for extracting DNA of the present invention developed for accomplishing the foregoing objects is explained as follows. The method comprises of:
(1) a process for pretreatment having;
   a procedure for obtaining fragments by using a whole sample of organism of hard tissue, by cutting the sample, or by crushing the sample, and
   a subsequent procedure of decalcification by adding an EDTA aqueous solution thereto,
(2) a process for dissolution having;
   a procedure for adding a buffer solution of a solubilizer, which dissolves a complex of DNA-protein in the sample of organism, and a proteolytic enzyme to a solution of (1) to dissolve a complex of DNA-protein,
(3) a process for extraction having;
   a procedure for adding a saturated phenol buffer including tris(hydroxymethyl)aminomethane hydrochloride or an aqueous solution including sodium iodide and isopropanol to the solution of the complex of (2) for deproteinization, and then separating an extract of DNA,
(4) a process for purification having;
   a subsequent process for purification of DNA by passing the extract of DNA of (3) through a column.

According to the method, the sample of the organism is used in whole or as a fragment that is cut or crushed even wider than powder. This allows the method to accomplish a precise determination of the only desired DNA of the sample of organism compared with prior methods for extracting DNA in which a thoroughly powdered sample is used.

With regard to a method for extracting DNA, examples of the hard tissue are a tooth and a bone.

It is preferable that the procedure for obtaining the fragments comprises of:
using the whole tooth, cutting the tooth or the bone into pieces of 0.1 to 0.5cm thick, or using crushed grains of at least about 5mm wide to obtain the fragments that include no powder.

In the process for the pretreatment, the EDTA aqueous solution may include a surface-active agent.

The decalcification is performed by the EDTA aqueous solution. Simultaneously, the surface-active agent in the solution properly advances the decalcification. Therefore, the process for the pretreatment is completed in short time and is capable of extracting only DNA efficiently and sufficiently.

It is preferable that the surface-active agent is an ion surface-active agent.

It is furthermore preferable that the ion surface-active agent is sodium dodecyl sulfate (SDS).

In the process for the pretreatment, it is preferable that concentration of the surface-active agent in the EDTA aqueous solution is ranging from 0.1 to 0.5 weight %.

In the process for the pretreatment, it is preferable that the procedure of decalcification is performed by soaking the sample in the EDTA aqueous solution at 20 to 60 °C for 2hours to 2days.

In the process for the pretreatment, it is furthermore preferable that the procedure of decalcification is performed by soaking the sample in the EDTA aqueous solution and the surface-active agent at 37 to 60 °C for 2hours to 2days.

In the process for the pretreatment, the sample of organism may be beforehand cleaned by a procedure for cleaning.

In the process for the pretreatment, it is preferable that the procedure for cleaning comprises of:
washing the sample of organism with an aqueous solution of a neutral detergent, water and then ethanol, and drying the sample.

In the process for the dissolution, it is preferable that the solution of DNA is prepared by adding the proteolytic enzyme to the solution of the complex and then keeping the solution at 37 to 65 °C for 0.5 to 6 hours.

It is preferable that the process for the extraction comprises of:
adding the phenol buffer,
separating aqueous sepernatant liquid, and
when a volume is redundant, concentrating by ultrafiltration to separate the extract of DNA.

In the process for the purification, it is preferable that the column is a silica menbrane column, a column filled with silica beads, or a centrifugal procedure using sodium iodide.

According to the method for extracting DNA of the present invention, it accomplishes no contamination of impurities such as adherents to the sample of the organism and protein that obstructs the DNA analysis.

Without precipitation by ethanol, in which much DNA is lost, it also accomplishes to extract DNA simply and efficiently with excellent reproducibility. This method does not require proficiency and is capable of extracting DNA from not only small or large amount of the sample but also plural samples.

As the before-mentioned "Forensic Sciences, 44(3), p.264-272, (2003)" mentions, a rate of detecting all locus was 27.2% when STR 13-locus was detected from degraded DNA after a tooth or a bone was powdered by prior methods.

By contrast, when DNA is extracted from a tooth of remains of war dead of 60years age by the method of the present invention, a rate of detecting all locus is 70%. Moreover, a rate of detecting all or partial locus is approximately 80% or more. Therefore, this extractive method is significantly more superior to the prior methods in which a tooth or a bone is powdered.

Because no powdery sample of organism is used in this method, decomposed DNA or impurities from the powdery sample do not contaminate desired DNA. Therefore, the purity of extracted DNA is sufficient.

In the procedure of the decalcification of a degraded sample, when the EDTA aqueous solution and the surface-active agent in the process for the pretreatment are used, working hours are shorter in comparison with the decalcification using EDTA aqueous solution without the surface-active agent. Furthermore, efficiency of extracting DNA is improved.

Additionally, this method accomplishes no mistake for the DNA analysis because high quality DNA is obtained from a small amount of the precious sample.

### Brief Description of Drawings

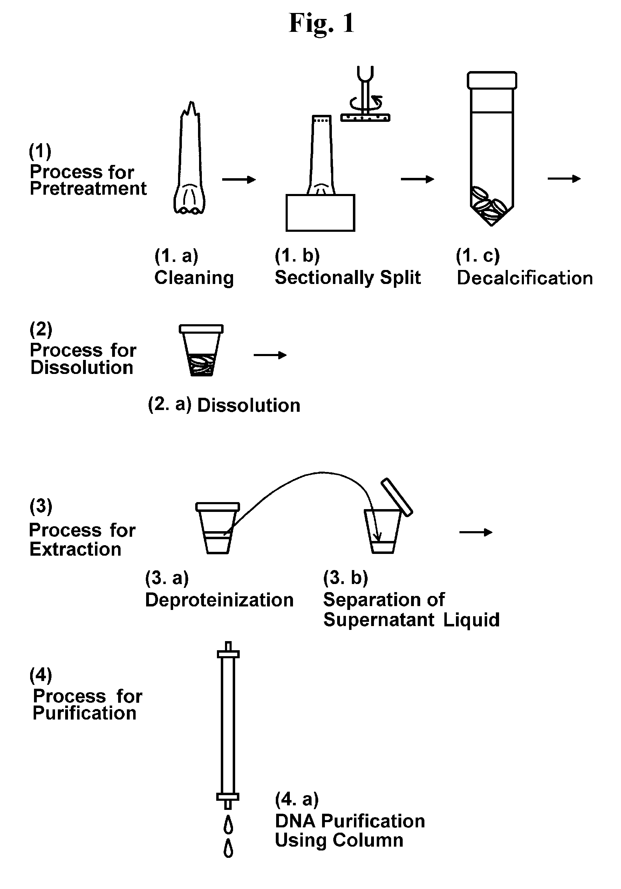

Fig. 1 is an outline of the processes of the method for extracting DNA of the present invention.

### Best Mode for Carrying Out the Invention

Hereunder, embodiments of the present invention are explained in detail, but the range of the present invention is not limited to these embodiments.

An example of the embodiment is explained referring to Fig.1. DNA is obtained through the following processes (1) to (4).

### (1) the process for the pretreatment

As a sample of organism, a tooth or a bone of a living human, or remain is used. The sample of organism is cleaned up by the procedure for cleaning such as wiping, washing and so on. Concretely, the procedure is performed as follows. The tooth or the bone is scrubbed with a brush soaked in a neutral detergent to remove the adherents from the surface thereof. The neutral detergent thereof is washed away with water, and then the tooth or the bone is washed with ethanol and dried off with a dryer or neutral convection. (See Fig. 1 (1.a))

The sample of the organism is cut or crushed to be used as grains or pieces having about 0.5cm size. If the sample of the organism is the tooth, it is used in whole. With these procedures, the contamination of undesired DNA from other tissue is completely prevented in comparison with using the powdery sample.

When the sample is the tooth, a tooth root thereof is cut to sectional splits having 0.1 to 0.2 cm thick, and 4 to 6 pieces of the fragments having about 60 to 120 mg are respectively obtained from one tooth. When the sample is the bone, a part having medulla is cut to fragments having 0.15 to 0.2 cm thick and 0.5 cm squares, and having about 60 to 120 mg are respectively obtained. In both cases, it is enough to use 1 to 4 pieces of the fragments as the sample of the organism. Thus, all remnants are allowed to be conserved. (See Fig. 1 (1.b))

Then the sample is soaked and incubated in the EDTA aqueous solution at 20 to 60 °C for 2hours to 2days in order to decalcify for removing constituent of calcium. It is preferable that soaking time and temperature are properly controlled according to its volume, shape or inferior level of the sample of the organism. It is furthermore preferable that the soaking temperature is ranging from 37 to 60 °C and the soaking time is within 24 hours. Also, it is preferable that the concentration of the EDTA aqueous solution is ranging from 0.2 to 0.5 M and is furthermore preferable that the concentration thereof is 0.5 M and pH thereof is 8.0. (See Fig. 1 (1.c))

### (2) the process for the dissolution

The buffer solution of the solubilizer is added to the decalcified sample of organism to dissolve the complex of DNA-protein. The buffer solution of the solubilizer includes 0.5 to 2.5 weight % of a detergent, and example of this solution is an aqueous solution of 100mM of sodium chloride, 10 mM of Tris hydrochloride and as the detergent 2 weight % of SDS.

Then the proteolytic enzyme is added to the solution of the complex, and the solution is maintained at 37 to 65 °C for 0.5 to 6 hours, if necessary with stirring. The proteolytic enzyme, which dissolves the protein in the complex, is not limited but is preferably proteinase K. (See Fig. 1 (2.a))

### (3) the process for the extraction

The saturated phenol buffer (pH 8.0) including 10mM of Tris hydrochloride and 1mM of EDTA is added to the solution of the complex of (2) in order to remove the protein or the decomposed protein. An example of the phenol buffer is TE saturated phenol that is a trade name and available from Nacalai Tesque Inc. (See Fig. 1 (3.a))

Then the solution is performed with centrifuge separation, and the aqueous phase including DNA is separated from supernatant liquid. When a volume of the aqueous phase is redundant, it may be concentrated with ultrafiltration of a centrifugation method. For example, Centricon 30 that is a trade name and available from Amicon Inc. is used for this method. With these procedures, the extract of DNA is separated. (See Fig. 1 (3.b))

### (4) the subsequent process for the purification

The extract of DNA is passed through a silica menbrane column for purification to obtain DNA. An example of the silica menbrane column is an attached column in QIAamp DNA Blood Mini Kit that is a registered trademark and available from QIAGEN. (See Fig. 1 (4.a))

Incidentally, the EDTA aqueous solution and SDS may be used instead of the EDTA aqueous solution used in the process for the pretreatment in the before-mentioned paragraph (1). On this occasion, it is preferable that the sample is incubated in the solution including EDTA and SDS at 37 to 60 °C for 2hours to 2days in order to decalcify for removing constituent of calcium. Also, it is preferable that incubating time and temperature are properly controlled according to its volume, shape or inferior level of the sample of the organism and is furthermore preferable that the incubating time is within 24 hours. It is also preferable that the concentration of EDTA aqueous solution is ranging from 0.2 to 0.5 M and is furthermore preferable that the concentration thereof is 0.5 M and pH thereof is 8.0. The concentration of the aqueous solution of SDS is preferably ranging from 0.1 to 0.5 weight %. (See Fig. 1 (1.c))

The column filled with the silica beads may be used instead of the silica menbrane column. An example of the column filled with the silica beads is MagExtractor that is used with magnetic silica beads and is a registered trademark and available from TOYOBO Co., LTD. It may be phenol-chloroform method, although the yield thereof is somewhat decreased.

Hereunder, Examples of the method for extracting DNA from actual samples are explained.

### (Example 1)

A tooth was scrubbed with a brush soaked in a neutral detergent to remove the adherents from the surface thereof. Sterilized distilled-water was then squirted thereto to wash the neutral detergent away. After that, ethanol was squirted thereto and then dried off with a dryer swiftly.

The tooth was put onto a wooden cubic base having each side of about 3 cm. 1 or 2 drops of an instant adhesive: aron alpha, which is a registered trademark and available from TOAGOSEI CO., LTD., were dropped into a clearance between the tooth and the base in order to fasten them together. In a clear plastic sheet made from vinyl polymer, a region of tooth root was cut to fragments having 0.1 cm or thicker with a discotic grinder to quarry 1 to 6 disc pieces. By lifting up and shaking the disc piece, the pieces attached no powder were obtained.

These 1 to 6 pieces were inserted into a tube for culture having 50 mL volume. 30 to 50 mL of 0.5 M EDTA aqueous solution (pH 8.0), which is a trade name and available from Wako Pure Chemical Industries, Ltd., was added to the tube, and then SDS was added thereto. SDS was regulated as 0.1 weight % in the solution. It was incubated at 37 to 50 °C for 12 hours to decalcify. The pieces of the tooth were moved to another tube for culture having 2 mL volume and washed with 700 µL of 0.5 M EDTA aqueous solution (pH 8.0). The EDTA aqueous solution in the tube was sucked out with a pipette.

As a buffer solution of a solubilizer, 100 to 200 µL of a resolvable solution including 10 mM of Tris hydrochloide, 100mM of sodium chloride and 2 weight % of SDS, and, as proteolytic enzyme, 20 to 40 µL of 20 mg/mL of proteinase K solution, which is a trade name and available from Wako Pure Chemical Industries, Ltd., were added to the pieces of the tooth in the tube. They were incubated with stirring at 50 °C for 1 to 2 hours, and then with additional 20 to 30 µL of proteinase K solution, they were incubated with stirring at 50 °C for 1 to 2 hours.

After it was confirmed that the half or more of the each piece of the tooth was dissolved in the tube, saturated phenol TE solution, which is a trade name and available from Wako Pure Chemical Industries, Ltd., as same as the volume thereof was added thereto. For 10 to 20 times, it was gently mixed by turnover. Then it was performed with centrifuge separation under 13000 r.p.m. for 5 minutes. A supernatant thereof was moved to the other tube for culture having 2 mL volume. When a volume of an aqueous phase thereof was redundant, it was concentrated with ultrafiltration using Centricon 30, which is a trade name and available from Amicon Inc., until 100 to 300 µL. Consequently, an extract of DNA was obtained.

DNA was purified from the extract of DNA by using QIAamp DNA Blood Mini Kit, which is a registered trademark and available from QIAGEN, according to its operational manual. Incidentally, for the final elution of DNA, 50 µL of AE buffer attached in QIAamp DNA Blood Mini Kit, which is a registered trademark and available from QIAGEN, was used.

When the obtained DNA was estimated by Polymerase Chain Reaction (PCR) and electrophoresis, it was proved that the obtained DNA included no impurity.

### (Example 2)

A tooth was scrubbed with a brush soaked in a neutral detergent to remove the adherents from the surface thereof. Sterilized distilled-water was then squirted thereto to wash the neutral detergent away. After that, ethanol was squirted thereto and then dried off with a dryer swiftly.

The whole tooth was inserted into a tube for culture having 50 mL volume. To 30 to 50 mL of 0.5 M EDTA aqueous solution (pH 8.0), which is a trade name and available from Wako Pure Chemical Industries, Ltd., SDS was added and regulated as 0.1 weight %, and then this solution was added to the tube. It was incubated at 37 to 50 °C for 12hours to decalcify. The pieces of the tooth were moved to another tube for culture having 2 mL volume and washed with 700 µL of 0.5 M EDTA aqueous solution (pH 8.0). The EDTA aqueous solution in the tube was sucked out with a pipette.

As a buffer solution of a solubilizer, 100 to 200 µL of a resolvable solution including 10 mM of Tris hydrochloide, 100mM of sodium chloride and 2 weight % of SDS, and, as proteolytic enzyme, 20 to 40 µL of 20 mg/mL of proteinase K solution, which is a trade name and available from Wako Pure Chemical Industries, Ltd., were added to the pieces of the tooth in the tube. They were incubated with stirring at 50 °C for 1 to 2hours, and then with additional 20 to 30 µL of proteinase K solution, they were incubated with stirring at 50°C for 1 to 2hours.

After it was confirmed that color of the solution became pale yellow and the tooth was dissolved in the tube, saturated phenol TE solution, which is a trade name and available from Wako Pure Chemical Industries, Ltd., as same as the volume thereof was added thereto. For 10 to 20 times, it was gently mixed by turnover. Then it was performed with centrifuge separation under 13000 r.p.m. for 5 minutes. A Supernatant thereof was moved to the other tube for culture having 2 mL volume. When a volume of an aqueous phase thereof was redundant, it was concentrated with ultrafiltration using Centricon 30, which is a trade name and available from Amicon Inc., until 100 to 300 µL. Consequently, an extract of DNA was obtained.

DNA was purified from the extract of DNA by using QIAamp DNA Blood Mini Kit, which is a registered trademark and available from QIAGEN, according to its operational manual. Incidentally, for the final elution of DNA, 50 µL of AE buffer attached in QIAamp DNA Blood Mini Kit, which is a trade name and available from QIAGEN, was used.

When the obtained DNA was estimated by Polymerase Chain Reaction (PCR) and electrophoresis, it was proved that the obtained DNA included no impurity.

### Industrial Applicability

The method for extracting DNA of the present invention is used for extracting DNA from a tooth or a bone as a hard tissue obtained from a remain of war dead or of ancient monument, and from dead body at a disaster and of a missing person. With this extractive method of the present invention, quality DNA can be extracted. Therefore, it accomplishes to obtain a sufficient data in forensic DNA analysis and biological genetic research.

## Claims

1. A method for extracting DNA comprising of:
(1) a process for pretreatment having;
a procedure for obtaining fragments by using a whole sample of organism of hard tissue, by cutting the sample, or by crushing the sample, and
a subsequent procedure of decalcification by adding an aqueous solution including ethylenediamine tetraacetic acid (EDTA) thereto,
(2) a process for dissolution having;
a procedure for adding a buffer solution of a solubilizer, which dissolves a complex of DNA-protein in the sample of organism, and a proteolytic enzyme to a solution of (1) to prepare a solution of a complex of DNA-protein,
(3) a process for extraction having;
a procedure for adding a saturated phenol buffer including tris(hydroxymethyl)aminomethane hydrochloride or an aqueous solution including sodium iodide and isopropanol to the solution of the complex of (2) for deproteinization, and then separating an extract of DNA,
(4) a process for purification having;
a subsequent process for purification of DNA by passing the extract of DNA of (3) through a column.

2. The method for extracting DNA according to claim 1, wherein the hard tissue is a tooth or a bone.

3. The method for extracting DNA according to claim 2, wherein the procedure for obtaining the fragments comprises of:
using the whole tooth, cutting the tooth or the bone into pieces of 0.1 to 0.5cm thick, or using crushed grains of at least about 5mm wide to obtain the fragments that include no powder.

4. The method for extracting DNA according to claim 1, wherein the EDTA aqueous solution in the process for the pretreatment includes a surface-active agent.

5. The method for extracting DNA according to claim 4, wherein the surface-active agent is an ion surface-active agent.

6. The method for extracting DNA according to claim 5, wherein the ion surface-active agent is sodium dodecyl sulfate (SDS).

7. The method for extracting DNA according to claim 4, wherein concentration of the surface-active agent in the EDTA aqueous solution is ranging from 0.1 to 0.5 weight %.

8. The method for extracting DNA according to claim 1, wherein the procedure of decalcification in the process for the pretreatment comprises of:
soaking the sample in the EDTA aqueous solution at 20 to 60 °C for 2hours to 2days.

9. The method for extracting DNA according to claim 4, wherein the procedure of decalcification in the process for the pretreatment comprises of:
soaking the sample in the EDTA aqueous solution and the surface-active agent at 37to 60 °C for 2hours to 2days.

10. The method for extracting DNA according to claim 1, wherein the sample of organism in the process for the pretreatment is beforehand cleaned by a procedure for cleaning.

11. The method for extracting DNA according to claim 10, wherein the procedure for cleaning in the process for the pretreatment comprises of:
washing the sample of organism with an aqueous solution of a neutral detergent, water and then ethanol, and drying the sample.

12. The method for extracting DNA according to claim 1, wherein the solution of DNA in the process for the dissolution is prepared by adding the proteolytic enzyme to the solution of the complex, and then keeping the solution at 37 to 65 °C for 0.5 to 6 hours.

13. The method for extracting DNA according to claim 1, wherein the process for the extraction comprises of:
adding the saturated phenol buffer and
separating aqueous sepernatant liquid

14. The method for extracting DNA according to claim 1, wherein the column in the process for purification is a silica menbrane column, a column filled with silica beads, or a centrifugal procedure using sodium iodide.
